# EUROPEAN PATENT APPLICATION

(11) **EP 3 296 269 A1**
(43) Date of publication of application: **21.03.2018**
(21) Application number: 16189092.6
(22) Date of filing: 16.09.2016
(51) Int. Cl.: C02F 1/72, A61L 2/232, C09D 5/14, C09D 5/16

(54) **HETEROPOLYOXOMETALATES**

(71) Applicant: POM Patentverwaltungs GbR, 35619 Braunfels (DE)
(72) Inventor: ARTAL LAHOZ, Carmen, 50007 Zaragoza (ES); BISCHOF, Andreas, 10407 Berlin (DE); BUÑUEL MAGDALENA, Miguel, 50017 Zaragoza (ES); LAZARO VILLAROYA, Guillermo, 50005 Zaragoza (ES); SANZ NAVAL, Javier, 50193 Zaragoza (ES); SCHEPERS, Klaus, 35619 Braunfels (DE); MISCHO, Horst, 54295 Trier (DE)
(74) Representative: Teipel, Stephan

(57) **Abstract**

The present invention relates to the use of heteropolyoxometalates for disinfecting purposes and in antimicrobial surfaces, paints or coatings.

## Description

The present invention relates to the use of heteropolyoxometalates for disinfecting purposes and in antimicrobial surfaces, paints or coatings.

In many industrial and domestic processes waste water is produced or fresh water is to be stored for later purposes. As essentially all water sources provide non-sterile water, in fresh water and in particular in waste water there is the risk of microbiological contamination during storage or processing.

Many ways are known in the art to avoid creation of microbiological contaminations, in particular biofilms, in containers for the storage of drinking water, waste water, surface water or similar liquids. Further, several methods are known to avoid microbiological contamination of surfaces, e.g. in hospitals or slaughterhouses. For example, usually the surfaces of containers are cleaned by heat treatment, e.g. by rinsing the surfaces or containers with hot liquids or gases, e.g. hot water or steam. Further, treatment by ozone or chlorine containing gases is known for disinfecting surfaces and containers and cleaning and removal of organic contaminations.

It is also known to apply ultraviolet (UV-) radiation to disinfect surfaces, containers, and even liquids. It is also known to add certain substances to the liquids, such as titanium dioxide, or to apply titanium dioxide to a surface for disinfecting or cleaning purposes. However, these surfaces and substances require UV-radiation for activating the disinfecting efficacy of titanium dioxide.

Further, it is known to apply silver ions or copper ions to solutions in order to reduce the microbiological contamination.

All these measures and procedures are disadvantageous as they waste a lot of energy, require high apparatus cost and costs of operation in order to be sufficiently effective, or rather expensive substances, such as silver ions or copper ions, have to be used, the latter having further disadvantages with regard to water and soil pollution. Further, using substances which are dangerous to handle, e.g. ozone or chlorine containing substances, as well as ultraviolet radiation, is disadvantageous and requires additional safety measures.

The use of polyoxometalates is known in the art for several purposes, e.g. in the general area of analytical chemistry (e.g. elemental analysis, electron staining), the use as catalysts including photo catalysts, in biochemistry for inhibiting electron transfer processes and as electron-dense and rigid components in the crystallization of biomolecules (e.g. ribosomes, leading to the 2009 Nobel Prize), and in medicine due to their antiviral and antitumor activity. The use of polyoxometalates as acid and oxidation catalysts is known in industry (e.g. for the hydration of olefins).

There is still a need in the art to provide an effective, environmentally friendly and cheap measurement to avoid microbial contamination of liquids and surfaces.

It has been surprisingly found that certain heteropolyoxometalates provide excellent antimicrobial efficiency when applied to surfaces, which are in contact with liquids, in particular with waste water, and thereby avoid microbiological contamination of surfaces and of the liquid and lead to a reduction of biofilm formation.

In particular, it has been found that certain heteropolyoxometalates based on phosphorous in combination with molybdenum or tungsten and at least one ammonium or phosphonium cation or at least one sulfonium cation as defined below show excellent antimicrobial activity, compared to other heteropolyoxometalates, in particular the known heteropolyoxometalates based on silicon. Surprisingly, the antimicrobial activity seems to be assured by the simple presence of oxygen in the air or dissolved in liquids, thus no activation of the heteropolyoxometalate is necessary, and no addition of activation agents.

Therefore, the present invention relates to the use of heteropolyoxometalates of the Formula (I), (II) or (III):

**A_{q+3}PV_{q}Z_{12-q}O₄₀,** **(I)**

**A₆P₂Z₁₈O₆₂,** **(II)**

or

**A₃PZ₄O₂₄** **(III)**

wherein
Z is selected from Mo or W,
index q = 0, 1, 2 or 3, and

A is selected from one or more cations and comprises at least one of the following Formulas (IV), (V) and (VI):

**R¹R²R³R⁴N⁺,** **(IV)**

**R¹R²R³R⁴P⁺,** **(V)**

and

**R¹R²R³S⁺** **(VI)**

wherein R¹, R², R³ and, if present, R⁴ are independently selected from hydrocarbons and H, provided that at least one of R¹, R², R³ and, if present, R⁴ is hydrocarbon and at least one of R¹, R², R³ and, if present, R⁴ is H,
for providing self-cleaning, stripping, disinfecting, self-sanitizing, biocidal, antimicrobial, antifouling, and/or deodorizing properties to at least part of a substrate or a surface of a substrate or to a coating or for decomposition and/or degradation of organic materials.

It is understood that, if due to the selection of the cation A in the heteropolyoxometalate the charge of the compound of the Formula (I), (II) or (III) should not be zero, then either the number of cations A can be reduced and/or the charge is balanced by one or more further cations and/or anions.

The present invention also relates to the use of a mixture of two or more differing heteropolyoxometalates of formula (I), (II) or (III), i.e. at least one parameter Z or q is selected differently for each anion of each cation A, or the same heteropolyoxometalate anion of formula (I), (II) or (III) is present together with at least two differing cations A.

The present invention in a certain embodiment also relates to the use of a mixture of several differing heteropolyoxometalates of formula (I), (II) or (III), i.e. at least one parameter Z or q is selected differently for each anion of each ammonium cation A, or the same heteropolyoxometalate anion of formula (I), (II) or (III) is present together with at least two differing ammonium cations.

Generally, polyoxometalates based on molybdenum (Mo) or tungsten (W) are known in the art, in particular as Keggin-type polyanions [XZ₁₂O₄₀]ⁿ⁻, wherein X is selected from P, Si, Ge, As, B or Al. It is also known that the Keggin-type polyoxometalates contain a central heteroatom (X), which can be e.g. phosphorus (P⁵⁺), silicon (Si⁴⁺), germanium (Ge⁴⁺), etc. Further, in the Keggin-type polyoxometalate based on molybdenum or tungsten one or more, in particular 1 to 3, molybdenum or tungsten atoms may be replaced by vanadium atoms (e.g. V⁵⁺). Another example of polyoxoanions is the Wells-Dawson species [X₂Z₁₈O₆₂]ⁿ⁻. This polyanion contains two heteroatoms (X), which can be phosphorus (P⁵⁺) or arsenic (As⁵⁺) and 18 molybdenum or tungsten atoms (Z). Further, in the Wells-Dawson-type polyoxometalate based on tungsten one or more, in particular 1 to 3, tungsten atoms may be replaced by vanadium atoms (e.g. V⁵⁺). Also several types of peroxotungstates are known. One of the most important ones is the Venturello polyoxoanion [PW₄O₂₄]³⁻, whose catalytic properties have been widely studied. The Venturello polyoxoanion consists of the central [PO₄]³⁻ group, which is connected to four {WO(O₂)₂} units. Generally, these polyoxometalate anions are known in the art, and the negative charge of the known anions is typically counterbalanced by cations like Li⁺, Na⁺, K⁺ or NH₄⁺ which provide solubility of the resulting salts in water.

The heteropolyoxometalates used in the present invention, on the other hand, were found to be less soluble or even insoluble in water. Thus, it was found that when A is selected as described above to counterbalance the negative charge of the certain heteropolyoxometalates, the heteropolyoxometalates, as a result, are rendered less soluble or even insoluble in water.

It was also surprisingly found that when A is selected as described above to counterbalance the charge of the heteropolyoxometalates, then the heteropolyoxometalates, as a result, are not only less soluble or even insoluble in water but also possess favorable stability, specifically thermal stability, in particular when the said cations are phosphonium cations. This thermal stability can be very advantageous, especially when the heteropolyoxometalates are included in such substrates as tiles, ceramics, enamels, glazes, coatings, or other materials that require high temperatures e.g. for firing and/or finishing.

In the heteropolyoxometalates used according to the present invention A is selected from one or more cations and comprises at least one cation selected from the group consisting of ammonium cations, phosphonium cations and sulfonium cations. These cations are used to counterbalance the negative charge of the heteropolyoxometalate anions. It is preferred that, more than one, more than two, or, if applicable, more than three, more than four, or even all cations are selected from the list above. If more than one cation is selected from the list, these may be the same or different.

In the heteropolyoxometalates used in the present invention the "heteroatom" is phosphorous. Preferred are the heteropolymolybdate derivatives of the fomula (I), wherein residue Z is molybdenum (Mo), and polytungstates of formula (III), wherein residue Z is tungsten (W).

In a preferred embodiment in the heteropolyoxometalate of formula (I) index q is 2 or 3, in particular 2. This means that in the heteropolyoxometalate based on molybdenum or tungsten, from the 12 molybdenum oxide or tungsten oxide subunits two or three are replaced by vanadium oxide subunits. In an especially preferred embodiment, the heteropolyoxometalate is of the formula (VII).

**A₅PV₂Z₁₀O₄₀,** **(VII)**

wherein Z is selected from Mo or W; A is selected from one or more cations and comprises at least one quaternary ammonium cation, quaternary phosphonium cation or tertiary sulfonium cation, the quaternary ammonium and phosphonium cation being preferred. Another preferred example is A₅PMo₁₀V₂O₄₀.

In another specifically preferred embodiment in the heteropolyoxometalate of formula (III) residue Z is W, i.e. the heteropolyoxometalate is A₃PW₄O₂₄.

In the heteropolyoxometalates used in the present invention, A is selected from one or more cations. The ammonium cation is of Formula (IV), the phosphonium cation is of Formula (V) and the sulfonium cation isof Formula (VI)

**R¹R²R³R⁴N⁺,** **(IV)**

**R¹R²R³R⁴P⁺,** **(V)**

**R¹R²R³S⁺** **(VI)**

wherein residues R¹, R², R³ and R⁴ can be independently selected. Preferably, residues R¹, R², R³ and R⁴ are independently selected from hydrocarbons including polymers. Preferably the hydrocarbons are C₁ to C₈₀ hydrocarbons, such as C₁ to C₂₀ hydrocarbons, more preferably C₁ to C₁₆ hydrocarbons, in particular C₂ to C₈ hydrocarbons, e.g. C₄ to C₆ hydrocarbons or C₄ to C₁₆ hydrocarbons. The hydrocarbons being preferably branched or straight, saturated or unsaturated alkyl groups, cycloalkyl groups, alkyloxyalkyl groups, aryl groups or heteroaryl groups. Examples are methyl, ethyl, propyl, butyl, pentyl, hexyl, heptyl, octyl, nonyl, decyl, undecyl, dodecyl, tridecyl, tetradecyl, pentadecyl, hexadecyl, heptadecy and octadecyl residues. Optionally, two of the residues R¹, R², R³ and, if present, R⁴ are part of a ring, e.g. together form a ring, optionally together with the central nitrogen, phosphor or sulfur atom. The ring may be saturated or unsaturated, such as aromatic.

The C1 to C80 (or C₁ to C₈₀) hydrocarbons encompass branched or straight, saturated or unsaturated, substituted or unsubstituted alkyl groups, aryl groups or heteroaryl groups. These residues thus can also be waxes or wax-like.

Examples for these C1 to C80 hydrocarbons include methyl, ethyl, propyl, butyl, pentyl, hexyl, heptyl, octyl, nonyl, decyl, undecyl, dodecyl, tridecyl, tetradecyl, pentadecyl, hexadecyl, heptadecy, octadecyl, nonadecyl, eicosanyl, C21 alkyl, C22 alkyl, C23 alkyl, C24 alkyl, C25 alkyl, C26 alkyl, C27 alkyl, C28 alkyl, C29 alkyl, C30 alkyl, C31 alkyl, C32 alkyl, C33 alkyl, C34 alkyl, C35 alkyl, C36 alkyl, C37 alkyl, C39 alkyl, C40 alkyl, C41 alkyl, C42 alkyl, C43 alkyl, C44 alkyl, C45 alkyl, C46 alkyl, C47 alkyl, C48 alkyl, C49 alkyl, C50 alkyl, C51 alkyl, C52 alkyl, C53 alkyl, C54 alkyl, C55 alkyl, C56 alkyl, C57 alkyl, C58 alkyl, C59 alkyl, C60 alkyl, C61 alkyl C62 alkyl, C63 alkyl, C64 alkyl, C65 alkyl, C66 alkyl, C67 alkyl, C68 alkyl, C69 alkyl, C70 alkyl, C71 alkyl, C72 alkyl, C73 alkyl, C74 alkyl, C75 alkyl, C75 alkyl, C76 alkyl, C77 alkyl, C78 alkyl, C79 alkyl, and C80 alkyl residues. These C1 to C80 hydrocarbons are preferably straight.

Preferred are C1 to C60 hydrocarbons including methyl, ethyl, propyl, butyl, pentyl, hexyl, heptyl, octyl, nonyl, decyl, undecyl, dodecyl, tridecyl, tetradecyl, pentadecyl, hexadecyl, heptadecy, octadecyl, nonadecyl, eicosanyl, C21 alkyl, C22 alkyl, C23 alkyl, C24 alkyl, C25 alkyl, C26 alkyl, C27 alkyl, C28 alkyl, C29 alkyl, C30 alkyl, C31 alkyl, C32 alkyl, C33 alkyl, C34 alkyl, C35 alkyl, C36 alkyl, C37 alkyl, C39 alkyl, C40 alkyl, C41 alkyl, C42 alkyl, C43 alkyl, C44 alkyl, C45 alkyl, C46 alkyl, C47 alkyl, C48 alkyl, C49 alkyl, C50 alkyl, C51 alkyl, C52 alkyl, C53 alkyl, C54 alkyl, C55 alkyl, C56 alkyl, C57 alkyl, C58 alkyl, C59 alkyl and C60 alkyl residues. These C1 to C60 hydrocarbons are preferably straight.

More preferred are C1 to C40 hydrocarbons including methyl, ethyl, propyl, butyl, pentyl, hexyl, heptyl, octyl, nonyl, decyl, undecyl, dodecyl, tridecyl, tetradecyl, pentadecyl, hexadecyl, heptadecy, octadecyl, nonadecyl, eicosanyl, C21 alkyl, C22 alkyl, C23 alkyl, C24 alkyl, C25 alkyl, C26 alkyl, C27 alkyl, C28 alkyl, C29 alkyl, C30 alkyl, C31 alkyl, C32 alkyl, C33 alkyl, C34 alkyl, C35 alkyl, C36 alkyl, C37 alkyl, C39 alkyl and C40 alkyl residues. These C1 to C40 hydrocarbons are preferably straight.

More preferred are C1 to C30 hydrocarbons including methyl, ethyl, propyl, butyl, pentyl, hexyl, heptyl, octyl, nonyl, decyl, undecyl, dodecyl, tridecyl, tetradecyl, pentadecyl, hexadecyl, heptadecy, octadecyl, nonadecyl, eicosanyl, C21 alkyl, C22 alkyl, C23 alkyl, C24 alkyl, C25 alkyl, C26 alkyl, C27 alkyl, C28 alkyl, C29 alkyl, and C30 alkyl residues. These C1 to C30 hydrocarbons are preferably straight.

More preferred are C2 to C25 hydrocarbons including ethyl, propyl, butyl, pentyl, hexyl, heptyl, octyl, nonyl, decyl, undecyl, dodecyl, tridecyl, tetradecyl, pentadecyl, hexadecyl, heptadecy, octadecyl, nonadecyl, eicosanyl, C21 alkyl, C22 alkyl, C23 alkyl, C24 alkyl, and C25 alkyl. These C2 to C25 are preferably straight.

Particularly preferred are C4 to C20 hydrocarbons including butyl, propyl, hexyl, heptyl, octyl, nonyl, decyl, undecyl, dodecyl, tridecyl, tetradecyl, pentadecyl, hexadecyl, heptadecy, octadecyl, nonadecyl, and eicosanyl residues. These C4 to C20 hydrocarbons are preferably straight.

In general, it is more preferable that at least one of R¹, R², R³, and, if present, R⁴ is a hydrocarbon with at least 4 carbons, such as e.g. butyl, pentyl, hexyl etc. It is more preferable that at least two of R¹, R², R³, and, if present, R⁴ is a hydrocarbon with at least 4 carbons, such as e.g. butyl, pentyl, hexyl etc..

It furthermore was found that, if the cation A in the heteropolyoxometalates described above contains a polymer residue or a heterocyclic residue, the obtained heteropolyoxometalate exhibits particularly preferred properties with respect to low water solubility and thermal stability as well as high antimicrobial effectivity. The invention therefore also relates to heteropolyoxometalates of the formula (I^{II}), (II^{II}) or (III^{II}) as well as the use of these heteropolyoxometalates for providing self-cleaning, stripping, disinfecting, self-sanitizing, biocidical, antimicrobial, antifouling, and/or deodorizing properties to at least part of a substrate or a surface of a substrate or to a coating or for decomposition and/or degradation or organic materials

**A'_{q-3}PV_{q}Z_{12-q}O₄₀,** **(I^{II})**

**A'₆P₂Z₁₈O₆₂,** **(II^{II})**

or

**A'₃PZ₄O₂₄** **(III^{II})**

wherein
Z is selected from Mo or W,
index q = 0, 1, 2 or 3, and
A' is selected from one or more cations and comprises at least one cation selected from

**R¹R²R³R⁴N⁺,** **(IV)**

**R¹R²R³R⁴P⁺,** **(V)**

**R¹R²R³S⁺** **(VI)**

wherein residues R¹, R², R³ and, if present, R⁴ are independently selected from H, polymers and C₁ to C₈₀ hydrocarbons, and, if none of R¹, R², R³ and, if present, R⁴ is polymer, at least two of R¹, R², R³ and, if present, R⁴ form a ring together with the nitrogen, phosphor or sulfur atom, provided that at least one of R¹, R², R³ and, if present, R⁴ is other than H and at least one of R¹, R², R³ and, if present, R⁴ is H. Herein, R¹, R², R³ and R⁴ are preferably as defined above.

The ring formed together with the nitrogen, phosphor or sulfur atom may, for example, be a three, four, five, six or seven-membered ring which may be saturated or unsaturated and which may contain one or more further heteroatoms, such as oxygen, nitrogen, phosphor or sulfur atoms. If the ring is unsaturated and if a double bond is present at the nitrogen, phosphor or sulfur atom of the ammonium, phosphonium or sulfonium cation, then one of R¹, R² R³ or R⁴ may be absent. Examples of suitable rings are aziridinium, thiiranium, azetidinium, thietium, pyrrolidinium, tetrathydrothiophenium, pyrrolium, thiophenium, piperidinium, tetrahydrothiopyranium, pyridinium, thiopyrylium, hexamethyleniminium, hexamethylensulfidium, azatropilidenium, thiotropilidenium, pyrazolium, imidazolium, benimidazolium, imidazolinium, indolium, chinolinium, isochinolinium, purinium, pyrimidinium, oxazolium, thiazolium and thiazinium as well as the phosphorous analogs of these ring systems.

The rings may be substituted by one or more hydrocarbon residues, in particular C₁ to C₁₂ alkyl or aryl (in particular phenyl) C₁ to C₆ alkyl residues. Suitable cations containing a ring are for example 1-butyl-3-methylimidazolium, 1-butyl-2,3-dimetylimidazolium, 1-methyl-3-octylimidazolium, 1-hexadecyl-3-methylimidazolium, 1,3-didecyl-3-methylimidazolium and 1-benzyl-3-methylimidazolium.

Suitable polymers are for example polymers comprising cationic side chains, such as phosphonium-containing cationic poly(styrene) polymers, hydroxy exchange membranes comprising ammonium hydroxide or phosphonium hydroxide functional groups, poly(vinylamine) derivatives as described for example in EP 0 580 078 A1, polymeric phosphonium ionomers, as described for example in WO 94/10214, poly(alkyl- and aryl)p-phenoxy-phenylsulfonium salts, poly(acrylamide-co-diallyl-dimethylammonium) and poly(diallyldimethylammonium).

In one embodiment of the present invention, the heteropolyoxometalates are supported, in particular supported on oxides such as magnesium, aluminum, silicon, titanium or cerium oxide, preferably silicon oxide (SiO₂). The oxides, in particular the silicon oxide, preferably has a high surface area, e.g. at least about 150 m²/g. The weight ratio of heteropolyoxometalates of the present invention to the supporting oxide, in particular silicon oxide, is about 10:1 to 1:100, preferably 1:1 to 1:10, e.g. about 1:2.

The heteropolyoxometalates of the formula (I), (II) and (III) can be prepared according to known processes.

One typical process for the preparation of the heteropolyoxometalates of the formula (I) and (II) comprises the steps of:
a) dissolving a salt comprising [PZ₁₁O₃₉]⁷⁻ anions, when q=1, a salt comprising [PZ₁₀O₃₆]⁷⁻ anions , when q=2, a salt comprising [PZ₉O₃₄]⁹⁻ anions, when q=3, or a salt comprising [PO₄]¹⁻ anions, optionally a salt comprising VO₃⁻ anions, and a salt comprising ZO₄²⁻ anions
   wherein
   Z is selected from Mo or W,
   in an aqueous solvent, preferably water, to obtain a solution,
b) adding an acid in order to decrease the pH of the solution obtained after step a), and
c) adding a compound containing at least one cation selected from the group consisting of quaternary ammonium cations, quaternary phosphonium cations and tertiary sulfonium cations.

One typical process for the preparation of the heteropolyoxometalates of the formula (III) comprises the steps of:
a) dissolving a salt or acid comprising [PZ₁₂O₄₀]³⁻, wherein Z is Mo or W in an aqueous solution of hydrogen peroxide, to obtain a solution,
b) adding an acid in order to decrease the pH of the solution of step a), and
c) adding a compound containing at least one cation selected from the group consisting of quaternary ammonium cations, quaternary phosphonium cations and tertiary sulfonium cations.

The heteropolyoxomolybdates or the heteropolyoxotungstates [PZ₉₋₁₁O₃₄₋₄₀]^{y-}, as used in step a) of the process for the preparation of the heteropolyoxometalates of the formula (I) and (II), and [PZ₁₂O₄₀]³⁻, as used in step a) of for the preparation of the heteropolyoxometalates of the formula (III), can be obtained as known in the art, (e.g. as described in Holleman-Wiberg, Lehrbuch der Anorganischen Chemie, 101st edition, pages 1467-1469; M. T. Pope, Heteropoly and Isopoly Oxometalates, Springer, 1983; or Huheey et al., Anorganische Chemie, Prinzipien von Struktur und Reaktivität, 4. Auflage, 2012, chapter 16.2 etc). In particular, aqueous solutions comprising molybdate and tungstate anions as well as the corresponding heteroatom oxoanions or acids, i.e. phosphoric acid H₃PO₄, or the respective anion PO₄³⁻, respectively, is acidified, and the heteropolyoxomolybdates or heteropolyoxotungstates are obtained by crystallization or chemical precipitation.

In step a) of the process for the preparation of the heteropolyoxometalates of the formula (I) VO₃⁻ anions are provided in solution, and a corresponding salt comprising the lacunary (= vacant) heteropolyoxomolybdate or heteropolyoxotungstate anions [PZ₉₋₁₁O_{34-39]}^{y-}, wherein Z is Mo or W, or are added to obtain a solution in an aqueous solvent, preferably water.

In steps b) of the processes for the preparation of the heteropolyoxometalates of the formula (I), (II) or (III) a suitable acid, preferably a mineral acid, e.g. hydrochloric acid or sulfuric acid is added to decrease the pH of the solution obtained as a result of the steps a).

In steps c) of the processes for the preparation of the heteropolyoxometalates of the formula (I), (II) or (III) at least one compound containing a cation A as described above or a compound able to provide or release the cation A as described above is added. Preferably, the ammonium cation containing compound comprises a compound of formula (IV'), the phosphonium cation containing compound comprises a compound of formula (V') and the sulfonium cation containing compound comprises a compound of formula (VI')

**(R¹R²R³R⁴N)ₚX,** **(IV')**

**(R¹R²R³R⁴P)ₚX,** **(V')**

**(R¹R²R³S)ₚX** **(VI')**

wherein residues R¹, R², R³ and R⁴ are as defined above, X is sulfate or a halogen ion, preferably chloride or bromide, and p is 2 if X is sulfate and p is 1 if X is a halogen ion.

The heteropolyoxometalates of formula (I), (II) or (III) are obtained as a gel or precipitate, which is typically washed several times with H₂O and may be dried to obtain a powder.

Optionally, the heteropolyoxometalate may be brought into any suitable shape by dissolving the gel or powder in a suitable organic solvent, e.g. ethanol, and drying the resulting solution to obtain a corresponding gel or powder. This way, heteropolyoxometalates may be mixed to prepare the heteropolyoxometalates comprising at least two different cations, in particular ammonium cations or at least two different heteropolxyoxometalate anions as described above.

The present invention furthermore relates to a substrate comprising a heteropolyoxometalate as defined above, or a mixture thereof, wherein the substrate preferably is selected from the group consisting of a polymer body, a paint, a varnish, a coating, an ink, glass fibers or an inorganic oxide material.

A polymer body, as referred to herein, is any object comprising at least one polymer. The polymer body can e.g. be selected from the group consisting of: containers for storage of drinking water (e.g. in the beverage industry), waste water, or surface water; containers for waste decomposition; containers for water purification; heating systems; medical equipment; ship, boat, and roof coverings; roof tiles, indoor tiles, outdoor tiles; bathroom equipment; toilets, portable toilets; fibers and non-wovens; technical textiles, activewear fabrics; rain water sewers; exterior façades, elements of façades; tubing; furniture; water filters; air conditioning systems; air filters; safety protection masks; respirators; automotive air conditioning systems; humidifiers; disinfection sprays; air dryers; wall paints; packaging.

The term "paint", as used herein, refers to a liquid composition which upon administration to a substrate in a thin layer converts into solid, colored film. A paint, as referred to herein, also comprises lacquers. The term "paint" thus refers to the liquid composition before administration to a substrate and to the solid, colored film obtained after administration of the liquid paint composition to the substrate, and the skilled person will understand from the context, which state of the paint is meant. The color of the paint is achieved by colorants or pigments comprised in the paint.

The term "varnish", as used herein, refers to a liquid composition which upon administration to a substrate in a thin layer converts into a solid, colorless film. The term "varnish" thus refers to the liquid composition before administration to a substrate and to the solid, colorless film obtained after administration of the liquid varnish composition to the substrate, and the skilled person will understand from the context, which state of the varnish is meant.

The term "coating", as used herein, is any liquid composition that upon administration to a substrate in a thin layer converts into a solid film for decorative or protective purposes. The term "coating" thus refers to the liquid composition before administration to a substrate and to the solid film obtained after administration of the liquid coating composition to the substrate, and the skilled person will understand from the context, which state of the coating is meant.

The term "ink", as used herein, is a liquid composition containing pigments or dyes that upon administration to a substrate converts into a colored film for illustrating and/or lithographic purposes.

These paints, varnishes, coatings and inks typically comprise polymers selected from the group consisting of acrylate resins, polyurethane resins, silicon resins, polyester resins, alkyd resins, epoxy resins, phenolic resins, and urea or amine based resins.

In a preferred embodiment of the present invention, the substrate comprises a thermoplastic, an elastomer, a thermoplastic elastomer, a duroplast, or a mixture thereof.

The term "thermoplastic", as used herein, refers to a polymer that becomes pliable or moldable above a specific temperature and solidifies upon cooling. Examples for thermoplastic polymers include but are not limited to polyacrylates, acrylonitrile-butadiene-styrenes, polyamides such as nylon, polyacetic acid, polybenzimidazole, polycarbonate, polyether sulfone, polyetherether ketone, polyetherimide, polyethylene, polyphenylene oxide, polyphenylene sulfide, polypropylene, polystyrene, polyvinylchloriode, polyethyleneterephthalate, polyurethane, polyester and polytetrafluoroethylene (e.g. Teflon).

The term "elastomer", as used herein, refers to a polymer with viscoelasticity (having both viscosity and elasticity). Examples for elastomers include but are not limited to unsaturated rubbers such as natural polyisoprene (natural rubber), synthetic polyisoprene, polybutadiene, chloroprene rubber, butyl rubber, styrene-butadiene rubber, (hydrogenated) nitrile rubber, saturated rubbers such as ethylene propylene rubber, ethylene propylene diene rubber, epichlorohydrin rubber, polyacrylic rubber, silicone, silicone rubber, fluorosilicone rubber, fluoro- and perfluoroelastomers, and ethylene-vinyl acetate.

The term "thermoplastic elastomer", as used herein, refers to a class of copolymers or a physical mix of polymers which consists of materials with both thermoplastic and elastomeric properties. Examples for thermoplastic elastomers include but are not limited to styrenic block copolymers, polyolefin blends, elastomeric alloys, thermoplastic polyurethanes, thermoplastic copolyester, and thermoplastic polyamides.

The term "duroplast", as used herein, refers to a polymer which is no longer pliable after curing. Examples for duroplasts include but are not limited to aminoplasts, phenoplasts, epoxy resins, polyacrylates, polyurethanes, polyesters, urea formaldehyde resins, melamine formaldehyde resins, and phenol formaldehyde resins.

In a more preferred embodiment of the present invention, the substrate comprises a polymer selected from the group consisting of polypropylene, polyethylene, polyethyleneterephthalate, polyester, polyamide, polyurethane, polyacrylate, polycarbonate, polystyrene, polyimides, polymethacrylates, polyoxoalkylenes, poly(phenylene oxides), polyvinylesters, polyvinylethers, polyvinylidene chloride, acrylonitrile-butadiene-styrene, natural and synthetic polyisoprene, polybutadiene, chloroprene rubber, styrene-butadiene rubber, tetrafluoroethylene, silicone, acrylate resins, polyurethane resins, silicone resins, polyester resins, alkyd resins, epoxy resins, phenolic resins, and urea or amine based resins, or a mixture thereof.

In a further preferred embodiment, the substrate comprises the heteropolyoxometalate or the mixture thereof in an amount of 0.01 to 90 wt.-%, based on the total weight of the substrate, more preferably in an amount of 0.02 to 85 wt.-%, 0.05 to 80 wt.-%, 0.1 to 75 wt.-%, 0.2 to 70 wt.-%, 0.3 to 65 wt.-%, 0.4 to 60 wt.-%, 0.5 to 55 wt.-% and particularly preferably in an amount of 1 to 50 wt.-%, each based on the total weight of the substrate. The surface of a substrate wherein this amount of heteropolyoxometalate is present, is particularly suitable for imparting antimicrobial properties to the surface of the substrate and reduces, preferably inhibits, at the same time the growth of a biofilm on the surface of the substrate. Moreover, since the heteropolyoxometalates as described herein have a low solubility in water, they are not washed out of the substrate upon exposure to water and moisture which guarantees a long term efficacy of the heteropolyoxometalate in providing antimicrobial properties to the surface of the substrate and reducing, preferably inhibiting, the growth of a biofilm.

The heteropolyoxometalates as described herein can be incorporated into a substrate as described above, i.e. into a polymer body, a paint, a varnish or a coating, by means of techniques known to the person skilled in the art.

The incorporation of the heteropolyoxometalate into a polymer body can e.g. be achieved by mixing the heteropolyoxometalate with the polymer in order to obtain a dispersion or a compound of heteropolyoxometalate and polymer. Said dispersion or compound can then e.g. be submitted to injection molding or extrusion for forming the polymer body.

Alternatively, the heteropolyoxometalate can be mixed with the monomers before polymerization is carried out. After polymerization, the resulting polymer can e.g. be submitted to compounding processes, pressureless processing techniques (e.g. casting, dipping, coating, foaming) or compression molding, rolling and calendaring, extrusion, blow molding or injection molding processes or drawing, thermoforming or printing for forming the polymer body.

The polymer body as described herein can also be obtained by drylaid, airlaid, spunlaid/meltblown or wetlaid processes, in particular if the polymer body is for use as fiber material or non-woven material.

The incorporation of the heteropolyoxometalate into a paint can e.g. be achieved by mixing the heteropolyoxometalate, which e.g. can be used as a powder, with the liquid paint composition in order to obtain a suspension of the heteropolyoxometalate and the liquid paint composition. Said suspension can be stored before application of the paint in its intended use.

The incorporation of the heteropolyoxometalate into a varnish can e.g. be achieved by mixing the heteropolyoxometalate, which e.g. can be used as a powder, with the liquid varnish composition in order to obtain a suspension of the heteropolyoxometalate and the liquid varnish. Said suspension can be stored before application of the varnish in its intended use.

The incorporation of the heteropolyoxometalate into a liquid coating composition can e.g. be achieved by mixing the heteropolyoxometalate, which e.g. can be used as a powder, with a coating solution in order to obtain a suspension of the heteropolyoxometalate and the liquid coating solution. Said suspension can be stored before usage, i.e. application of the coating solution in its intended use.

The substrate comprising the heteropolyoxometalate as described herein can be used for self-cleaning, stripping, disinfecting, self-sanitizing, biocidal, antimicrobial, antifouling, and/or deodorizing purposes and/or for the decomposition and/or degradation of organic materials.

In particular, the substrate comprising the heteropolyoxometalate as described herein can be used for the disinfection of an aqueous media by means of incorporating the substrate into an aqueous media such as waste water, surface water, drinking water etc.. The heteropolyoxometalate comprised in the substrate then activates molecular oxygen and/or hydrogen peroxide comprised in the aqueous media, thereby generating reactive oxygen species (ROS) such as the hyperoxide anion (O₂⁻), which are emitted from the surface of the substrate being surrounded by the aqueous media, thereby evolving an antimicrobial and disinfecting effect in the surrounding aqueous media. The oxidized heteropolyoxometalate in the surface of the substrate, in particular the cationic moiety thereof, reacts with the negatively charged cell membrane of microorganisms contained in the surrounding aqueous media by means of electron transfer, thereby regenerating the heteropolyoxometalate, which subsequently activates molecular oxygen comprised in the aqueous medium. The substrates for this application include filter materials, non-wovens, polymers, glas fibers or inorganic oxide materials comprising the heteropolyoxometalates of the present invention, and which are provided with a large surface.

The present invention relates to the use of the heteropolyoxometalates as described above for self-cleaning, stripping, disinfecting, self-sanitizing, biocidal, antimicrobial, antifouling, and/or deodorizing (odor removing) purposes. Said purposes can be characterized by the ability to self-clean and/or strip by degrading, decomposing and stripping organic materials (e.g. oxidation of impurities such as grease, paint and other residues; avoiding formation of biofilms, etc.); by the ability to deter, render harmless, destroy and/or exert a controlling effect on all forms and/or parts of microbial life (e.g. bacteria, viruses, fungi, spore forms, etc.); and/or by the ability to remove and/or reduce offensive and/or unpleasant scents.

The above described qualities can be used to modify substrates. Wherein one or more parts (e.g. materials, additives, surface layers, inner layers, exterior, interior, etc.) of the said substrate comprise a heteropolyoxometalate as described above, and/or one or more of the surfaces (e.g. functional surfaces) of the said substrate are at least partially coated with surface layers, paints, coatings, cleaners and/or stripping agents, wherein said surface layers, paints, coatings, cleaners (e.g. oxidizing cleaners) and/or stripping agents comprise a heteropolyoxometalate. The substrates as described above can be selected from the group consisting of: containers for storage of drinking water (e.g. in the beverage industry), waste water, or surface water; containers for waste decomposition; containers for water purification; hospitals, medical equipment, slaughterhouses, ships, boats, roof coverings, roof tiles, indoor tiles, outdoor tiles, kitchen rooms, sinks, wash closets, toilets, portable toilets, ceramics, polymers, fibers, rain water sewers, exterior façades, elements of façades, pools, pumps, tubing, technical textiles, activewear fabrics, paper, wood, apparatus for air and water purification, apparatus for soil decontamination, window glass (e.g. self-cleaning windows), mirrors (e.g. anti-fog coatings for mirrors and/or glass), filter materials such as nonwovens, respirator masks, air filters, such as air-conditioner filters, water filters, and activated-carbon filters for water and/or air purification.

The heteropolyoxometalates may further be used for decomposition and/or degradation of organic materials (e.g. plastics, organic polymers, etc), such as for recycling, reuse, and/or disposal purposes.

The heteropolyoxometalates may further be used for pulp and/or fiber bleaching and/or treatment.

## Claims

1. Use of a heteropolyoxometalate of the Formula (I), (II) or (III)
**A_{q+3}PV_{q}Z_{12-q}O₄₀,** **(I)**
**A₆P₂Z₁₈O₆₂,** **(II)**
or
**A₃PZ₄O₂₄** **(III)**
wherein
Z is selected from Mo or W,
index q = 0, 1, 2 or 3, and
A is selected from one or more cations and comprises at least one cation of the following Formulas (IV), (V) and (VI):
**R¹R²R³R⁴N⁺,** **(IV)**
**R¹R²R³R⁴P⁺,** **(V)**
and
**R¹R²R³S⁺** **(VI)**
wherein R¹, R², R³ and, if present, R⁴ are independently selected from hydrocarbons and H, provided that at least one of R¹, R², R³ and, if present, R⁴ is hydrocarbon and at least one of R¹, R², R³ and, if present, R⁴ is H,
for providing self-cleaning, stripping, disinfecting, self-sanitizing, biocidal, antimicrobial, antifouling, and/or deodorizing properties to at least part of a substrate or a surface of a substrate or to a coating or for decomposition and/or degradation of organic materials.

2. The use of claim 1, wherein in the heteropolyoxometalate of Formula (I) Z is Mo and q = 2.

3. The use of claim 1, wherein in the heteropolyoxometalate of Formula (III) Z is W.

4. The use of any one of claims 1 to 3, wherein in the heteropolyoxometalate A is selected from ammonium cations and phosphonium cations, preferably A comprises at least two different of these cations.

5. The use of any one of claims 1 to 3, wherein in the heteropolyoxometalate A comprises at least one ammonium cation.

6. The use of claim 5, wherein in the heteropolyoxometalate A comprises at least two different ammonium cations.

7. The use of any one of claims 1 to 6, wherein in the cation of Formula (IV), (V) or (VI)residues R¹, R², R³ and, if present, R⁴ are independently selected from H, polymers and C₁ to C₈₀ hydrocarbons, and optionally at least two of the residues R¹, R², R³ and, if present, R⁴ are part of a ring or form a ring together with the nitrogen, phosphor or sulfur atom, provided that at least one of R¹, R¹, R³ and, if present, R⁴ is hydrocarbon and at least one of R¹, R², R³ and, if present, R⁴ is H.

8. The use of any one of claims 1 to 6, wherein in the cation of Formula (IV), (V) or (VI) residues R¹, R², R³ and, if present, R⁴ are independently selected from H, substituted or unsubstituted alkyl, alkoxyalkyl or cycloalkyl groups that may contain at least one heteroatom selected from the group consisting of O, S, N and P, wherein the alkyl, alkoxyalkyl and cycloalkyl group preferably has at least 5 carbon atoms.

9. A heteropolyoxometalate of the Formula (I^{II}), (II^{II}) or (III^{II})
**A'_{q+3}PV_{q}Z_{12-q}O₄₀,** **(I^{II})**
**A'₆P₂Z₁₈O₆₂,** **(II^{II})**
or
**A'₃PZ₄O₂₄** **(III^{II})**
wherein
Z is selected from Mo or W,
index q = 0, 1, 2 or 3, and
A' is selected from one or more cations and comprises at least one cation selected from
**R¹R²R³R⁴N⁺,** **(IV)**
**R¹R²R³R⁴P⁺,** **(V)**
**R¹R²R³S⁺** **(VI)**
wherein residues R¹, R², R³ and, if present, R⁴ are independently selected from H, polymers and C₁ to C₈₀ hydrocarbons, and, if none of R¹, R², R³ and, if present, R⁴ is polymer, at least two of R¹, R², R³ and, if present, R⁴ form a ring together with the nitrogen, phosphor or sulfur atom, provided that at least one of R¹, R², R³ and, if present, R⁴ is other than H and at least one of R¹, R², R³ and, if present, R⁴ is H.

10. A substrate comprising a heteropolyoxometalate as defined in any of the preceding claims, or a mixture thereof, wherein the substrate preferably is selected from the group consisting of a polymer body, a paint, a varnish, a coating, an ink, glass fibers and an inorganic oxide material.

11. Substrate according to claim 10, wherein the substrate further comprises a polymer selected from the group consisting of thermoplastics, elastomers, thermoplastic elastomers, and duroplasts, preferably selected from polypropylene, polyethylene, polyethyleneterephthalate, polyester, polyamide, polyurethane, polyacrylate, polycarbonate, polystyrene, polyimides, polymethacrylates, polyoxoalkylenes, poly(phenylene oxides), polyvinylesters, polyvinylethers, polyvinylidene chloride, acrylonitrile-butadiene-styrene, natural and synthetic polyisoprene, polybutadiene, chloroprene rubber, styrene-butadiene rubber, tetrafluoroethylene, silicone, acrylate resins, polyurethane resins, silicone resins, polyester resins, alkyd resins, epoxy resins, phenolic resins, and urea or amine based resins, or a mixture thereof.

12. Substrate according to claim 10 or 11, wherein the substrate comprises the heteropolyoxometalate in an amount of 0.01 to 90 wt.-%, based on the total weight of the substrate.

13. Substrate according to any of claims 10 to 12, wherein the substrate is a polymer body obtainable by compounding processes, pressureless processing techniques (e.g. casting, dipping, coating, foaming) or compression molding, rolling and calendaring, extrusion, blow molding or injection molding processes or drawing, thermoforming or printing or drylaid, airlaid, spunlaid/meltblown or wetlaid processes.

14. Use of a substrate as defined in any of claims 10 to 13 for self-cleaning, stripping, disinfecting, self-sanitizing, biocidal, antimicrobial, antifouling, and/or deodorizing purposes and/or for the decomposition and/or degradation of organic materials.

15. Use of a substrate as defined in any of claims 10 to 13 for the disinfection of an aqueous medium.
